# EUROPEAN PATENT SPECIFICATION

(11) **EP 1 951 337 B1**
(45) Date of publication and mention of the grant of the patent: **01.08.2018**
(21) Application number: 06799812.0
(22) Date of filing: 25.09.2006
(51) Int. Cl.: A61M 1/34, A61M 1/16, A61M 1/36

(54) **EXTRACORPOREAL BLOOD CLEANING**
EXTRAKORPORALE BLUTREINIGUNG
HEMODIALYSE EXTRACORPORELLE

(30) Priority: 17.10.2005 SE 0502294; 24.10.2005 US 596822 P
(43) Date of publication of application: 06.08.2008
(73) Proprietor: Gambro Lundia AB, 220 10 Lund (SE)
(72) Inventor: STERNBY, Jan, S-226 52 Lund (SE); WIESLANDER, Anders, S-227 38 Lund (SE); KJELLSTRAND, Per, S-247 36 Södra Sandby (SE); LINDÉN, Torbjörn, S-290 11 Linderöd (SE)
(74) Representative: Sweden SHS IP Office
(86) International application number: PCT/SE2006/050349
(87) International publication number: WO 2007/046757

(56) References cited:
- EP-A1- 1 101 501
- EP-A2- 0 264 695
- WO-A1-01/58496
- WO-A1-01/94349
- WO-A1-2004/091694
- WO-A2-01/62314
- US-A- 5 942 112
- US-A1- 2001 051 106

## Description

### THE BACKGROUND OF THE INVENTION AND PRIOR ART

The present invention relates generally to extracorporeal blood cleaning. More particularly the invention relates to an apparatus according to the preamble of claim 1 and a method according to the preamble of claim 27.

The human body consists of approximately 60% water - a level which is important to maintain for survival. While it is unproblematic to provide the body with new water, disposal of surplus water is a major problem in renal patients. One task of the normal kidney is to remove superfluous fluid from the blood, such as water, urea and other waste products. The resulting urine is transferred to the bladder and finally leaves the body during urination. The kidney's second task is to regulate for example the balance of electrolytes and acid and base. With malfunctioning kidneys, disorders may develop in most major body organs, a syndrome called uremia. If uremia remains untreated, it will lead to death. Uremia is treated either by kidney transplantation or some form of extracorporeal blood cleaning, e.g. hemodialysis, hemofiltration or hemodiafiltration.

Extracorporeal blood cleaning may also be employed in connection with blood donation to a blood bank. Namely, when storing whole blood for future use, for instance in surgery, it is of utmost importance that the blood has a highest possible quality.

Today, the vast majority of all extracorporeal blood cleaning is performed on whole blood. Such processing is very good at removing undesired substances (i.e. toxins) that are dissolved in the blood plasma. In general, diffusive treatments have a higher efficiency than convective treatments for substances with a relatively low molecular weight, and for larger substances the convective transport is more efficient.

In a renal patient, the water concentration of urea is considered to be the same inside and outside of the cells. When urea is removed through dialysis from the plasma water outside the cell a concentration difference occurs across the cell. This, in turn, causes urea to diffuse out of the cell, and thus clean the cell. The transport of urea is quick because the cell wall is highly permeable to urea. In fact, the net result is that urea is removed almost as well from inside the cells as from the plasma water. However, there exist a number of substances that are not so readily available for removal by means of a dialysis-based strategy. One reason for this may be that the substances are mainly residing inside the cells, and thus also within the red blood cells. If the cell membrane is relatively impermeable with respect to the substances, the above-described equilibration of concentrations between inside and outside of the cell becomes complicated, or even impossible. Creatinine, phosphate and potassium constitute examples of substances for which the cell membrane permeability is relatively low.

Another reason for a slow dialysis removal rate may be that the substance to be removed is bound to proteins, such as albumin, residing in the plasma. Namely, the removal rate in dialysis is proportional to the concentration of the substance in the plasma water. If a large part of the substance is bound to proteins, the ratio between the removal rate and the total amount present in the blood will be smaller than for non-protein bound substances. This is due to the fact that the removal rate is proportional to the dissolved concentration, and the total amount includes both the protein bound substances and the dissolved substances. Thus, the time required for the removal increases. Bilirubin, which is a breakdown product from the normal turnover of red blood cells, is one example of a protein bound substance. Hippuric acid and paracresol represent other toxin examples of this type.
One approach to improve the dialysis removal rate is to separate the blood cells from the plasma. For instance, the published international patent application WO01/62314 discloses a hemodialysis system in which whole blood is processed in a centrifuge to separate the cells from the plasma fluid and foreign substances. The plasma fluid and foreign substances are then conveyed away from the centrifuge where the foreign substances are removed from the plasma. Thereafter, the plasma is combined with the cells to a resulting cleaned whole blood. However, there is no cleaning treatment of the cells. This means that any toxins therein remain also in the resulting whole blood. Further, EP 0 264 695 A2 describes a method for filtration of blood taken from the patient. The method is carried out step-wise, using a first membrane system which is permeable to medium-sized molecules. The filtrate obtained is subjected to a second filtration using a second membrane system which is permeable only to small molecules. The retentate is removed. The filtered blood transferred from the first membrane system is dialysed, and the dialysed blood is combined with the permeate of the second membrane system and is returned to the patient without addition of substituate. The device suitable for this purpose consists of a housing with three hollow-fibre membrane chambers, with flow connection between the chambers. The published international patent application WO99/20377 describes an ultradialyzer for removing toxins from a patient's blood, wherein the blood is divided into two portions. One portion contains mainly blood cells, and the other portion essentially contains the plasma part of the blood. The concentrated blood cells are led to an inner section of a dialysis apparatus and the plasma is directed to an outer section. Dialysis fluid is circulated in a countercurrent direction on an outer side of a membrane for both the inner and outer sections. Back filtration occurs into the concentrated blood cells in the inner section, as well as dialysis by diffusion. In the outer section, the plasma is treated by means of dialysis, which is facilitated in the absence of blood cells. However, the cleaning efficiency with respect to urea in the inner section is reduced due to the higher blood cell and protein concentrations. Hence, the overall blood cleaning efficiency is reduced. Nevertheless, this method implies treating both fractions by the same method, i.e. dialysis consisting of diffusion, ultrafiltration and convection.

### SUMMARY OF THE INVENTION

The object of the present invention is therefore to alleviate the efficiency problems discussed above, and thus accomplish an improved solution for all kinds of extracorporeal blood cleaning.

According to one aspect of the invention, the object is achieved by the initially described apparatus, wherein the first processing branch includes at least one first processing unit, which is adapted to remove cell-bound substances from the blood cells according to a first cleaning process and thus produce resulting washed blood cells. The second processing branch includes at least one second processing unit, which is adapted to remove toxins bound on proteins within the plasma and/or toxins dissolved in the plasma according to a second cleaning process, which is different from the first cleaning process.

An important advantage attained by this design is that each of the first and second processes can be tailored for an optimal cleaning of the blood cells and the plasma respectively. For instance, processing strategies that are per se very efficient, however harmful to one of the blood fractions may be applied to the other fraction without causing any damage to the resulting whole blood. Moreover, certain treatments of the plasma fraction may be more difficult, or more expensive, to perform in the presence of cell fractions, and vice versa. Naturally, this processing vouches for a very high overall toxin removal rate.

It is worth noting that the proposed dividing-off of the first and second fractions from the incoming blood does not preclude that further fractions in addition to the above-mentioned two fractions are divided off from the incoming blood, and that also these fractions are treated separately. For example, the blood cells may be sub-divided into red cells and white cells, so that at least one of the different cell types can be treated by means of a process being optimized for this type of cells. Moreover, in the mixing of the cleaned fractions one or more of any such sub-fractions may be left out.

According to one preferred embodiment of this aspect of the invention, the first processing branch includes a convection unit, which is adapted to remove the cell-bound substances from within the blood cells of the first fraction by means of a concentration-based cleaning process that operates over the cell membranes by causing an increased convection due to osmotic forces. Thereby, the removal rate of any toxins residing in the blood cells can be enhanced. A high osmolarity (i.e. salt content) is known to trigger the complement system. Therefore, it is an advantage not to have a high osmolarity in all of the blood. By separating blood cells and plasma into different fractions it is possible to decrease the volume faced with the high osmolarity, so that the complement system is less activated. It is also rendered easier to restore the osmolarity of the combined fractions to normal levels after the treatment when only one fraction has a high osmolarity, e.g. by keeping the other fraction(s) at a low osmolarity.

According to another preferred embodiment of this aspect of the invention, the first processing branch includes a first drug-inserting unit, which is adapted to add at least one input substance to the first fraction so as to produce a resulting pre-processed first fraction. This is advantageous because hence the toxin extraction process from the pre-processed first fraction may be facilitated, for example by adding an input substance that is adapted to enhance a removal rate of toxins from within the blood cells.

According to yet another preferred embodiment of this aspect of the invention, the input substance is adapted to influence the cell membranes of the blood cells of the first fraction, e.g. by increasing the permeability of the ion channels in the membrane, and thereby increase the membranes' permeability with respect to at least one toxin. By performing this treatment on the first fraction containing blood cells, less of the needed substances are required than for treating an equivalent amount of whole blood. Hence, the treatment can be made more efficient and less expensive. With less substance added it is also easier to remove the substance after the treatment.

According to still another preferred embodiment of this aspect of the invention, the input substance contains Riboflavin. Consequently, the pre-processed first fraction is a Riboflavin-containing fluid. Moreover, the first processing branch includes a light-processing unit, which is adapted to receive the pre-processed first fraction, and illuminate the pre-processed first fraction to generate a resulting first cleaned fraction. The first cleaned fraction has a bacteria concentration, which is lower than a bacteria concentration in the pre-processed first fraction. Thus, a reliable and straightforward bacteria reduction stage is attained.

According to another preferred embodiment of this aspect of the invention, the first processing branch includes an ultrasound unit. This unit is adapted to receive the first fraction, and expose the first fraction to ultrasonic energy. As a result, the blood cells' membrane permeability increases with respect to at least one toxin. Naturally, this vouches for an improved removal rate for the toxins in question.

According to a further preferred embodiment of this aspect of the invention, the first processing branch includes an electroporing unit. This unit is adapted to receive the first fraction, and expose the first fraction to an electric field, so as to increase the permeability of the cell membranes of the blood cells in the first fraction with respect to at least one toxin. This constitutes an alternative, or a complement means to increase the toxin removal rate. Both the above-mentioned ultrasound and electric field treatments are relatively simple due to the comparatively small volume resulting from treating only one fraction, i.e. the first fraction containing blood cells. Namely, a small volume facilitates reaching high ultrasonic energy levels and high electric-field strength for the electroporation respectively.

According to another preferred embodiment of this aspect of the invention, the first processing branch includes a chemotherapy unit, which is adapted to receive the first fraction. The chemotherapy unit is also adapted to expose the first fraction to a cancer treatment drug, so as to reduce an amount of cancer-damaged cells among the blood cells in the first fraction. This treatment is advantageous in that relatively high doses of therapy (i.e. high drug concentrations) can be used without subjecting the patient to an elevated risk of side effects, since the drug concentration can be reduced again in connection with combining the first and second fractions before returning the whole blood to the patient. A cleaning unit may also be included in the processing chain before returning the blood to the patient.

According to yet another preferred embodiment of this aspect of the invention, the first processing branch includes a gas-induction unit, which is adapted to receive the first fraction. The gas-induction unit is also adapted to induce carbon dioxide into the first fraction, so as to alter an intracellular pH level of the blood cells in the first fraction, and thereby enable an efficient removal of uremic toxins. This embodiment is particularly advantageous if an alkalotic plasma fraction is used because then the resulting whole blood may have a physiological pH level.

According to still another preferred embodiment of this aspect of the invention, the second processing branch includes a dialysis unit, which is adapted to produce the second cleaned fraction based on a hemodialysis process, a hemofiltration process or a hemodiafiltration process. Namely, all these processes represent efficient blood plasma purification strategies.

According to yet another preferred embodiment of this aspect of the invention, the dialysis unit is adapted to operate with a dialysis fluid having a level of electrolytes significantly lower than the physiological levels of a human being. Thereby, large fluid amounts can be used at a low cost. Operating the process is also made easier, since no dosing calculation is required. Furthermore, the dialysis unit is preferably adapted to operate with a dialysis fluid, which has a temperature significantly lower than a normal body temperature of a human being (i.e. a cold dialysis fluid). Hence, the power consumption can be held relatively low.

According to another preferred embodiment of this aspect of the invention, the second processing branch includes an acid-level-adjustment unit, which is adapted to lower the pH level of the second fraction significantly. This will decrease the binding of toxins to proteins, and thus the removal of the toxins is increased. Normally, exceedingly low pH levels would be harmful to the blood cells. However, since here, only the plasma is affected, this strategy becomes much less problematic.

According to still another preferred embodiment of this aspect of the invention, the acid-level-adjustment unit is adapted to lower the pH level of the second fraction to approximately 3 - 5. Thereby good plasma purification can be realized.

According to a further preferred embodiment of this aspect of the invention, the second processing branch includes an acid-level-adjustment unit, which instead is adapted to increase the pH level of the second fraction to approximately 8 - 12, or more preferably to a level falling within the interval 9 - 11. Namely, some undesired substances are removed more efficiently in a basic environment.

According to yet another preferred embodiment of this aspect of the invention, the second processing branch includes a second drug-inserting unit, which is adapted to add at least one input substance to the second fraction. As a result, a pre-processed second fraction is produced. Preferably, the input substance is adapted to reduce a protein binding of at least one component of the plasma in the second fraction. In the absence of blood cells, the input substances can be selected relatively freely to attain a desired cleaning effect.

According to one further preferred embodiment of this aspect of the invention, the input substance (e.g. caffeine) is adapted to compete with albumin as a carrier of at least one toxin in the plasma of the second fraction. Thereby, the toxins will be inclined to bind to the input substance instead, which will produce a carrier-toxin complex that is much smaller than albumin, and can therefore be more easily removed. Moreover, it is desired that albumin is not removed.

According to another preferred embodiment of this aspect of the invention, the second processing branch includes a light-processing unit. This unit is adapted to receive the second fraction, and illuminate the second fraction to generate a resulting pre-processed second fraction. Here, the pre-processed second fraction has a concentration of at least one light-sensitive toxin, which is lower than a concentration of the at least one light-sensitive toxin in the second fraction. An important advantage attained by dividing off the blood cells from the plasma is that incident light can reach the light-sensitive toxin (e.g. bilirubin) in the plasma comparatively easily. Hence, the binding of the light-sensitive toxin to albumin can be broken up more efficiently.

According to still another preferred embodiment of this aspect of the invention, the second processing branch includes an adsorption unit. This unit is adapted to receive the second fraction, or a pre-processed fraction thereof, and adsorb at least one toxin in the plasma. As a result, the second cleaned fraction is produced. For example, the adsorption unit may include at least one positively charged adsorption zone, which is adapted to capture negatively charged toxins in the plasma of the second fraction. Alternatively, or as a complement thereto, the adsorption unit may include at least one adsorption column, which is adapted to reduce the concentration of at least one toxin in the plasma of the second fraction. Also these strategies benefit from the absence of blood cells because such cells tend to attach to the adsorbing surfaces, and thereby both risk to become damaged and reduce the function of the adsorbing surfaces.

According to yet another preferred embodiment of this aspect of the invention, the second processing branch includes an electrodialysis unit including a dialysis membrane. The electrodialysis unit is adapted to receive either the second fraction, or a pre-processed fraction thereof. The electrodialysis unit is adapted to hold the dialysis membrane at an electrical tension, so as to remove charged substances from the plasma, and as a result produce the second cleaned fraction. For example, this strategy is well suited to remove glycosylated proteins from the plasma, since these proteins alter their charge when being glycosylated. Glycosylated proteins are precursors to so-called advanced glycosylation end products (AGEs), a group of substances considered to be detrimental to patients. Having the blood cells separated from the plasma renders it possible to further increase the efficiency by using higher electrical currents, which would otherwise risk killing the blood cells. A lowered pH also assists to reverse the glycosylation. Thus, this embodiment of the invention is preferably combined with the above-described acid-level-adjustment unit. Furthermore, the electrodialysis of the plasma-containing second fraction may be simplified if the dialysis fluid contains no, or a relatively small amount of, electrolytes because these are affected by the electrical tension of the membrane.

According to a further preferred embodiment of this aspect of the invention, the second processing branch includes a heating unit, which is adapted to receive the second fraction. The heating unit is adapted to elevate the temperature of the plasma, so as to reduce an amount of bacteria therein, and as a result produce the second cleaned fraction. This processing is advantageous because with the blood cells removed, bacteria's higher sensitivity to heat and pressure variations can be used to eliminate the bacteria, for instance by increasing the pressure at a temperature of, say 40-50° C (i.e. an artificial fever). Consequently, this embodiment is suitable for treatment of patients suffering from sepsis.

According to yet another preferred embodiment of this aspect of the invention, the second processing branch includes an antioxidant-inducing unit, which is adapted to receive the second fraction. The antioxidant-inducing unit is also adapted to induce at least one antioxidant substance (e.g. Vitamin C, Vitamin E, N-acetylcystein, various catalases or superoxid dismutase) into the plasma. Namely, thereby an amount of free radicals in the plasma is reduced, and a resulting second cleaned fraction is obtained. This treatment is advantageous because it is simpler, less expensive and requires less amounts of the antioxidant substance than if whole blood was treated. Although it is true that also the intracellular pool of antioxidants, such as glutathion, can be positively influenced in the blood-cell-containing first fraction by treatments of drugs, these drugs, in order to be efficient, are different from the antioxidants relevant to add to the plasma. Therefore, the proposed separation is still highly advantageous.

According to another aspect the specification discloses the initially described method, wherein the processing of the first fraction involves removal of cell-bound substances from the blood cells of the first fraction according, such that the first cleaned fraction contains washed blood cells. The processing of the second fraction involves a second cleaning process, which is different from the first cleaning process, and this process removes toxins bound on proteins within the plasma and/or toxins dissolved in the plasma.
The advantages of this method, as well as the preferred embodiments thereof, are apparent from the discussion hereinabove with reference to the proposed apparatus.
Generally, the invention enables highly efficient, reliable and cost-effective extracorporeal blood cleaning for many purposes of which dialysis is one very important example.
Further advantages, advantageous features and applications of the present invention will be apparent from the following description and the dependent claims.

### BRIEF DESCRIPTION OF THE DRAWINGS

The present invention is now to be explained more closely by means of preferred embodiments, which are disclosed as examples, and with reference to the attached drawings.
- Figure 1: shows a block diagram over an apparatus for extracorporeal blood cleaning according to one embodiment of the invention, and
- Figure 2: shows a flow diagram which illustrates the general method as disclosed in this specification.

### DESCRIPTION OF PREFERRED EMBODIMENTS OF THE INVENTION

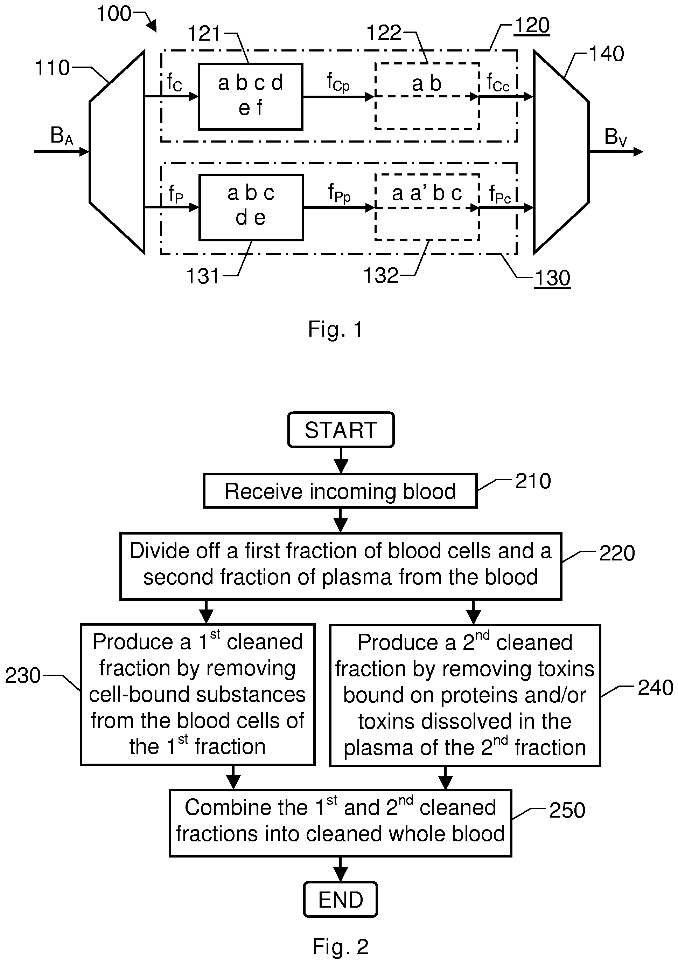

Figure 1 shows a block diagram over an apparatus 100 for extracorporeal blood cleaning according to one embodiment of the invention. The apparatus 100 includes a separation unit 110, a first processing branch 120, a second processing branch 130 and a mixing unit 140.
The separation unit 110 is adapted to receive incoming blood B_{A} and separate this blood into a first fraction f_{C} containing predominantly blood cells, and a second fraction f_{P} containing predominantly blood plasma. To this aim, the separation unit 110 may include a centrifuge, an ultrasound unit (e.g. of the type described in the document US 6,929,750) or a filter having a sufficiently high cutoff to allow the passage of albumin, however not blood cells. Preferably, however not necessarily, the filter is adapted to also hinder larger proteins, such as globulins, having a molecular weight above 100 kDa.
The first processing branch 120 includes at least one first processing unit 121, 122, which is adapted to receive the first fraction f_{C}, process the first fraction f_{C} according to a first cleaning process, and output a first cleaned fraction f_{Cc}. In the first cleaning process, the first processing unit(s) 121, and possibly 122, remove cell-bound substances from the blood cells, and produce resulting washed blood cells representing the first cleaned fraction f_{Cc}.

The second processing branch 130 includes at least one second processing unit 131, 132, which is adapted to remove toxins bound on proteins within the plasma and/or toxins dissolved in the plasma. The processing units 131, 132 operate according to a second cleaning process, which is different from the first cleaning process, to process the second fraction f_{P} and output a second cleaned fraction f_{Pc}.

The mixing unit 140 is adapted to receive the first and second cleaned fractions f_{Cc} and f_{Pc}, and physically combine these fractions. To this aim, the unit 140 preferably includes conventional fluid mixing means to accomplish a uniform cleaned whole blood B_{V} to be output from the apparatus 100.

Below, we will describe particular examples of processing units 121, 122, 131 and 132 that may be included in the processing branches 120 and 130 according to embodiments of the invention. The apparatus 100 may thus contain a number of specific units 121a, 121b, 121c, 121d, 121e, 121f, 122a, 122b, 131a, 131b, 131c, 131d, 131e, 132a 132a', 132b and 132c that are employed in combination with one another in one or more different configurations.

According to one embodiment, the first processing branch 120 includes a convection unit 121a, which is adapted to remove the cell-bound substances from within the blood cells of the first fraction f_{C} by means of a concentration-based cleaning process operating over the cell membranes. The convection unit 121a may employ a high ion concentration (e.g. salt based) to extract cell-bound substances like Creatinine from the blood cells in the first fraction f_{C}. Provided that salt, or similar, is added, an appropriate secondary processing unit 122b may need to be included in the processing branch 120 to remove the undesired substances along with any additives. Nevertheless, no extra processing may be required in the processing branch 120 if the plasma fraction in the second processing branch 130 is dialyzed against a fluid containing no, or only a small amount of, electrolytes. Then, the electrolyte content can be made correct after the mixing back to whole blood. Thus, either an appropriate processing is performed by the secondary processing unit 122b, or the treatment of the plasma fraction as such result in a treated plasma with a sufficiently low content of the salts in question, or similar.

According to one embodiment, either as a complement or as an alternative to the above, the first processing branch 120 includes a first drug inserting unit 121b, which is adapted to add at least one input substance to the first fraction f_{C}. As a result, a pre-processed first fraction f_{Cp} is produced. The pre-processed first fraction f_{Cp} may then be processed further to obtain the first cleaned fraction f_{Cc}. Preferably, the at least one input substance is adapted to enhance a removal rate of toxins from within the blood cells of the first fraction f_{C}. To this aim, it is desirable if the input substance is adapted to influence the cell membranes of the blood cells of the first fraction f_{C}, so as to increase the membranes' permeability with respect to at least one toxin. For example, the permeability of the ion channels in the membrane may be increased.

The input substance may be Riboflavin (i.e. vitamin B2), and the first processing branch 120 may contain a light processing unit 122a. Thus, here, the pre-processed first fraction f_{Cp} is a Riboflavin-containing fluid. Moreover, the light processing unit 122a is adapted to receive the pre-processed first fraction f_{Cp} and illuminate this fraction to generate a resulting first cleaned fraction f_{Cc}. The light processing unit 122a emits such light that after the illumination, the first cleaned fraction f_{Cc} has a bacteria concentration which is lower than a bacteria concentration in the pre-processed first fraction f_{Cp}.

According to one embodiment, either as a complement or as an alternative to the above, the first processing branch 120 includes an ultrasound unit 121c. This unit is adapted to receive the first fraction f_{C} and expose the fraction to ultrasonic energy, so as to increase the permeability of the cell membranes of the blood cells therein with respect to at least one toxin.

Alternatively, or as a complement thereto, the first processing branch 120 may include an electroporing unit 121d, which is adapted to receive the first fraction f_{C} and expose the first fraction f_{C} to an electric field. As a result, the permeability of the cell membranes of the blood cells in the first fraction f_{C} is increased with respect to at least one toxin.

Alternatively, or as yet another complement, the first processing branch 120 may include a chemotherapy unit 121e, which is adapted to receive the first fraction f_{C} and expose it to a cancer treatment drug. As a result, an amount of cancer-damaged cells is reduced among the blood cells in the first fraction f_{C}. Of course, this treatment is well suited for patients suffering from leukemia.

According to another embodiment of the invention, either as a complement or as an alternative to the above, the first processing branch 120 includes a gas-induction unit 121f, which is adapted to receive the first fraction f_{C}. The unit 121f then induces carbon dioxide into the first fraction f_{C}, so as to alter an intracellular pH level of the blood cells in the first fraction f_{C}. As a result, an efficient removal of uremic toxins is enabled.

In any of the above-described embodiments wherein the toxin removal rate from within the blood cells is enhanced, the first processing branch 120 may also include at least one secondary processing unit, e.g. a dialysis unit, to perform the actual removal of the toxins from the first fraction f_{C}.

According to one embodiment, either as a complement or as an alternative to the above, the second processing branch 130 includes a dialysis unit 132a. This unit is adapted to produce the second cleaned fraction f_{Pc} based on a hemodialysis process, a hemofiltration process or a hemodiafiltration process. Preferably, the dialysis unit 132a is adapted to operate with a dialysis fluid having a level of electrolytes that is significantly lower than the physiological levels of a human being. Namely, thereby large fluid amounts can be used at a low cost. Moreover, since no dosing calculation is required the practical operation of the process is rendered easier as well. As mentioned above, the osmolarity needs to be restored in the whole blood resulting from the combination of the cleaned fractions. This restoration may be performed with aid from the first fraction containing blood cells in case the second fraction has been treated with a high salt content. Alternatively, the restoration can be made at the end of the second processing branch 130 before combining the fractions in the mixing unit 140. This requires a considerably smaller amount of salt than if all of the treatment fluid has a physiological level of salt.

According to one preferred embodiment of the invention, the dialysis unit 132a is adapted to operate with a dialysis fluid that has a relatively low temperature being significantly lower than a normal body temperature of a human being (i.e. a cold dialysis fluid). This is advantageous with respect to power consumption and cost. However, the temperature needs to be restored if the whole blood is to be returned to a patient. Such a restoration may only partly be completed with respect to the blood cell containing fraction, since the blood cells cannot tolerate an elevated temperature. Nevertheless, again the restoration can be made at the end of the second processing branch 130, and this consumes much less power than performing the whole dialysis procedure at physiological temperatures, i.e. around 37° C.

According to one embodiment, either as a complement or as an alternative to the above, the second processing branch 130 includes an acid-level-adjustment unit 131a, which is adapted to lower the pH level of the second fraction f_{P} significantly. Preferably, the pH level is lowered to a value in the range from approximately pH 3 to approximately pH 5. According to preferred embodiments of the invention, the acid-level-adjustment unit 131a is adapted to infuse citric acid and/or hydrochloric acid to accomplish the desired decrease of the pH level. Lactic acid and acetic acid constitute other examples of acids that are possible to use in connection with dialysis, however these acids are less physiologic. Pyruvic acid could also be used.

As an alternative, citric acid may be used as a combined anticoagulant pH-level decreasing substance. However, this requires that the citric acid be infused directly when the blood is drawn from the patient in order for the anticoagulation to work.

In this embodiment, the second processing branch 130 may also include the dialysis unit 132a and a pH-level restoration unit 132a' adapted to raise the pH level to a normal value (e.g. pH 7,0 to pH 7,8) before passing the second cleaned fraction f_{Pc} to the mixing unit 140. The pH-level restoration unit 132a' may be adapted to infuse bicarbonate to attain this neutralizing effect.

To create a basic environment and thus remove certain undesired substances from the plasma, according to yet another embodiment of the invention, the second processing branch includes an acid-level-adjustment unit 131a, which is adapted to *increase* the pH level of the second fraction f_{P}. Preferably, the pH level is here raised to an interval ranging from approximately 8 to approximately 12, or more preferably to a level falling within the interval 9 - 11.

According to one embodiment, either as a complement or as an alternative to the above, the second processing branch 130 includes a second drug inserting unit 131b. The unit 131b is adapted to add at least one input substance to the second fraction f_{P}, and thus produce a resulting pre-processed second fraction f_{Pp}. The above-described dialysis unit 132a may then be used to remove the drug along with other toxins in the plasma.

Preferably, the input substance is adapted to reduce a protein binding of at least one component of the plasma in the second fraction f_{P}. Alternatively, the input substance, e.g. represented by caffeine, may be adapted to compete with albumin as a carrier of at least one toxin in the plasma of the second fraction f_{P}. Thereby, a secondary processing unit 132 may remove any protein-bound substances relatively easily from the second fraction f_{P}.

According to one embodiment, either as a complement or as an alternative to the above, the second processing branch 130 includes a light processing unit 131c. This unit is adapted to receive the second fraction f_{P} and illuminate the fraction to generate a resulting pre-processed second fraction f_{Pp}. This illumination may cause a breakdown of light-sensitive toxins into less tightly bound breakdown products, which are easier to remove. One example is bilirubin, which breaks down to form the more watersoluble lumirubin. According to one preferred embodiment of the invention, the light processing unit 131c is adapted to produce light containing relatively large amounts of energy around 450 nm (i.e. near the ultraviolet range), where bilirubin has its absorption peak. Namely, by illuminating the second fraction f_{P} with such light the bilirubin therein can be transformed into lumirubin. Lumirubin is water soluble, and may thus be removed efficiently by means of standard hemodialysis, hemofiltration or hemodiafiltration processing, for example implemented by the above-described dialysis unit 132a.

Nevertheless, the pre-processed second fraction f_{Pp} has a concentration of bilirubin, or other light-sensitive toxin (depending on the light used), which is lower than a concentration of the at least one light-sensitive toxin in the second fraction f_{P}.

According to one embodiment, either as a complement or as an alternative to the above, the second processing branch 130 includes an adsorption unit 132b. This unit is adapted to receive either the second fraction f_{P}, or a pre-processed fraction thereof f_{Pp} (e.g. produced by the acid-level-adjustment unit 131a, the second drug inserting unit 131b or the light processing unit 131c), and adsorb at least one toxin in the plasma. As a result, the adsorption unit 132b produces the second cleaned fraction f_{Pc}. The adsorption unit 132b, in turn, may include at least one positively charged adsorption zone, which is adapted to capture negatively charged toxins in the plasma of the second fraction f_{P}. Alternatively, the adsorption unit 132b may include at least one adsorption column, which is adapted to reduce the concentration of at least one toxin in the plasma of the second fraction f_{P}. As mentioned earlier, in this treatment it is advantageous not to have any blood cells present.

According to another embodiment of the invention, either as a complement or as an alternative to the above, the second processing branch 130 includes an electrodialysis unit 132c. This unit, in turn, has a dialysis membrane to which an electrical tension may be applied. Specifically, the electrodialysis unit 132c is adapted to receive the second fraction f_{P}, or a pre-processed fraction thereof f_{Pp}, and hold the dialysis membrane at an electrical tension. Thereby, charged substances are removed from the plasma and a resulting second cleaned fraction f_{Pc} is produced.

In another embodiment of the invention, the second processing branch 130 includes, either as a complement or as an alternative to the above, a heating unit 131d. This unit is adapted to receive the second fraction f_{P} and elevate the temperature of the plasma. Thereby, an amount of bacteria in the plasma may be reduced to produce the second cleaned fraction f_{Pc}. Preferably, the pressure of the plasma is increased at a temperature of 40-50° C.

According to yet another embodiment of the invention, the second processing branch 130 comprises an antioxidant inducing unit 131e. Also this unit may be combined with one or more of the above-described units. The antioxidant inducing unit 131e is adapted to receive the second fraction f_{P}, and induce at least one antioxidant substance into the plasma. As a result, an amount of free radicals in the plasma is reduced, such that it may constitute the second cleaned fraction f_{Pc}. Vitamin C, Vitamin E, N-acetylcystein, various catalases and/or superoxid dismutase may preferably be used as the antioxidant substance.

In order to sum up, the general method for extracorporeal blood cleaning according to the invention will be described below with reference to the flow diagram in figure 2.

A first step 210 receives incoming blood. A following step 220 divides off a first fraction from the blood, where the first fraction contains predominantly blood cells. The step 220 also divides off a second fraction containing predominantly blood plasma. Then, a step 230 processes the first fraction according to a first cleaning process by removing cell-bound substances from the blood cells to produce a first cleaned fraction containing washed blood cells. Preferably in parallel with the step 230, a step 240 processes the second fraction by removing toxins bound on proteins within the plasma and/or toxins dissolved in the plasma to produce a second cleaned fraction. The processing in the step 240 is different from the processing in the step 230. Furthermore, each of the steps 230 and 240 is adapted to optimize the treatment according to the characteristics of the respective fraction. Finally, a step 250 combines the first and second cleaned fractions into cleaned whole blood.

The term "comprises/comprising" when used in this specification is taken to specify the presence of stated features, integers, steps or components. However, the term does not preclude the presence or addition of one or more additional features, integers, steps or components or groups thereof.

The invention is not restricted to the described embodiments in the figures, but may be varied freely within the scope of the claims.

## Claims

1. An apparatus for extracorporeal blood cleaning comprising:
a separation unit (110) adapted to receive incoming blood (B_{A}) and from this blood divide off a first fraction (f_{C}) containing predominantly blood cells, and divide off a second fraction (f_{P}) containing predominantly blood plasma;
a first processing branch (120) adapted to receive the first fraction (f_{C}), process the first fraction (f_{C}) according to a first cleaning process, and output a first cleaned fraction (f_{Cc});
a second processing branch (130) adapted to receive the second fraction (f_{P}), process the second fraction (f_{P}), and output a second cleaned fraction (f_{Pc}); and
a mixing unit (140) adapted to receive the first and second cleaned fractions (f_{Cc}, f_{Pc}), combine these fractions (f_{Cc}, f_{Pc}), and output cleaned whole blood (B_{V}), wherein the second processing branch (130) comprises at least one second processing unit (131, 132) adapted to remove at least one of toxins bound on proteins within the plasma and toxins dissolved in the plasma according to a second cleaning process different from the first cleaning process, **characterized in that**
the first processing branch (120) comprises at least one first processing unit (121, 122) adapted to remove cell-bound substances from the blood cells, and produce resulting washed blood cells (f_{Cc}).

2. The apparatus according to claim 1, **characterized in that** the first processing branch (120) comprises a convection unit (121a) adapted to remove the cell-bound substances from within the blood cells of the first fraction (f_{C}) by means of a concentration-based cleaning process operating over the cell membranes.

3. The apparatus according to any one of the claims 1 or 2, **characterized in that** the first processing branch (120) comprises a first drug inserting unit (121b) adapted to add at least one input substance to the first fraction (f_{C}) so as to produce a resulting pre-processed first fraction (f_{Cp}).

4. The apparatus according to any one of the preceding claims, **characterized in that** the first processing branch (120) comprises an ultrasound unit (121c) adapted to:
receive the first fraction (f_{C}), and
expose the first fraction (f_{C}) to ultrasonic energy so as to increase the permeability of the cell membranes of the blood cells therein with respect to at least one toxin.

5. The apparatus according to any one of the preceding claims, **characterized in that** the first processing branch (120) comprises an electroporing unit (121d) adapted to:
receive the first fraction (f_{C}), and
expose the first fraction (f_{C}) to an electric field so as to increase the permeability of the cell membranes of the blood cells in the first fraction (f_{C}) with respect to at least one toxin.

6. The apparatus according to any one of the preceding claims, **characterized in that** the first processing branch (120) comprises a chemotherapy unit (121e) adapted to:
receive the first fraction (f_{C}), and
expose the first fraction (f_{C}) to a cancer treatment drug so as to reduce an amount of cancer-damaged cells among the blood cells in the first fraction (f_{C}).

7. The apparatus according to any one of the preceding claims, **characterized in that** the first processing branch (120) comprises a gas-induction unit (121f) adapted to:
receive the first fraction (f_{C}), and
induce carbon dioxide into the first fraction (f_{C}) so as to alter an intracellular pH level of the blood cells in the first fraction (f_{C}) to enable an efficient removal of uremic toxins.

8. The apparatus according to any one of the preceding claims, **characterized in that** the second processing branch (130) comprises a dialysis unit (132a) adapted to produce the second cleaned fraction (f_{Pc}) based on at least one of a hemodialysis process, a hemofiltration process and a hemodiafiltration process.

9. The apparatus according to any one of the preceding claims, **characterized in that** the second processing branch (130) comprises an acid-level-adjustment unit (131a) adapted to lower the pH level of the second fraction (f_{P}) significantly.

10. The apparatus according to claim 9, **characterized in that** the acid-level-adjustment unit (131a) is adapted to lower the pH level of the second fraction (f_{P}) to a level falling within an interval from approximately 3 to approximately 5.

11. The apparatus according to any one of the preceding claims, **characterized in that** the second processing branch (130) comprises an acid-level-adjustment unit (131a) adapted to increase the pH level of the second fraction (f_{P}) to a level falling within an interval from approximately 8 to approximately 12.

12. The apparatus according to any one of the preceding claims, **characterized in that** the second processing branch (130) comprises a second drug inserting unit (131b) adapted to add at least one input substance to the second fraction (f_{P}) so as to produce a resulting pre-processed second fraction (f_{Pp}).

13. The apparatus according to any one of the preceding claims, **characterized in that** the second processing branch (130) comprises a light-processing unit (131c) adapted to:
receive the second fraction (f_{P}), and
illuminate the second fraction (f_{P}) to generate a resulting pre-processed second fraction (f_{Pp}) having a concentration of at least one light-sensitive toxin which is lower than a concentration of the at least one light-sensitive toxin in the second fraction (f_{P}).

14. The apparatus according to any one of the preceding claims, **characterized in that** the second processing branch (130) comprises an adsorption unit (132b) adapted to:
receive the second fraction (f_{P}) or a pre-processed fraction thereof (f_{Pp}), and
adsorb at least one toxin in the plasma so as to produce the second cleaned fraction (f_{Pc}).

15. The apparatus according to claim 14, **characterized in that** the adsorption unit (132b) comprises at least one positively charged adsorption zone adapted to capture negatively charged toxins in the plasma of the second fraction (f_{P}).

16. The apparatus according to any one of the claims 14 or 15, **characterized in that** the adsorption unit (132b) comprises at least one adsorption column adapted to reduce the concentration of at least one toxin in the plasma of the second fraction (f_{P}).

17. The apparatus according to any one of the preceding claims, **characterized in that** the second processing branch (130) comprises an electrodialysis unit (132c) including a dialysis membrane, the electrodialysis unit (132c) is adapted to:
receive the second fraction (f_{P}) or a pre-processed fraction thereof (f_{Pp}), and
hold the dialysis membrane at an electrical tension so as to remove charged substances from the plasma, and produce the second cleaned fraction (f_{Pc}).

18. The apparatus according to any one of the preceding claims, **characterized in that** the second processing branch (130) comprises a heating unit (131d) adapted to:
receive the second fraction (f_{P}), and
elevate the temperature of the plasma so as to reduce an amount of bacteria therein, and produce the second cleaned fraction (f_{Pc}).

19. The apparatus according to any one of the preceding claims, **characterized in that** the second processing branch (130) comprises an antioxidant-inducing unit (131e) adapted to:
receive the second fraction (f_{P}), and
induce at least one antioxidant substance into the plasma so as to reduce an amount of free radicals therein, and produce the second cleaned fraction (f_{Pc}).

## Patentansprüche

1. Vorrichtung zur extrakorporalen Blutreinigung, umfassend:
eine Separationseinheit (110), die dazu angepasst ist, ankommendes Blut (B_{A}) aufzunehmen und von diesem Blut eine erste Fraktion (f_{C}) abzuteilen, die prädominant Blutzellen enthält, und eine zweite Fraktion (f_{P}) abzuteilen, die prädominant Blutplasma enthält;
einen ersten Verarbeitungszweig (120), der dazu angepasst ist, die erste Fraktion (f_{C}) aufzunehmen, die erste Fraktion (f_{C}) entsprechend einem ersten Reinigungsverfahren zu verarbeiten, und eine erste gereinigte Fraktion (f_{Cc}) auszugeben;
einen zweiten Verarbeitungszweig (130), der dazu angepasst ist, die zweite Fraktion (f_{P}) aufzunehmen, die zweite Fraktion (f_{P}) zu verarbeiten und eine zweite gereinigte Fraktion (f_{Pc}) auszugeben; und
eine Mischeinheit (140), die dazu angepasst ist, die erste und die zweite gereinigte Fraktion (f_{Cc}, f_{Pc}) aufzunehmen, diese Fraktionen (f_{Cc}, f_{Pc}) zu kombinieren und gereinigtes Vollblut (Bᵥ) auszugeben, wobei der zweite Verarbeitungszweig (130) mindestens eine zweite Verarbeitungseinheit (131, 132) umfasst, die dazu angepasst ist, mindestens eins von an Proteine innerhalb des Plasmas gebundene Toxine und im Plasma gelöste Toxine entsprechend einem zweiten Reinigungsverfahren, das sich von dem ersten Reinigungsverfahren unterscheidet, zu entfernen,
**dadurch gekennzeichnet, dass**
der erste Verfahrenszweig (120) mindestens eine erste Verarbeitungseinheit (121, 122) umfasst, die dazu angepasst ist, zellgebundene Substanzen aus den Blutzellen zu entfernen und resultierende gewaschene Blutzellen (f_{Cc}) zu produzieren.

2. Vorrichtung nach Anspruch 1, **dadurch gekennzeichnet, dass** der erste Verarbeitungszweig (120) eine Konvektionseinheit (121a) umfasst, die dazu angepasst ist, die zellgebundenen Substanzen aus den Blutzellen der ersten Fraktion (f_{C}) mithilfe eines konzentrationsbasierten Reinigungsverfahrens, das über die Zellmembranen arbeitet, zu entfernen.

3. Vorrichtung nach einem der Ansprüche 1 oder 2, **dadurch gekennzeichnet, dass** der erste Verarbeitungszweig (120) eine erste Medikamenteneinführeinheit (121b) umfasst, die dazu angepasst ist, der ersten Fraktion (f_{C}) mindestens eine Eingabesubstanz hinzuzufügen, um eine resultierende vorverarbeitete erste Fraktion (f_{Cp}) zu produzieren.

4. Vorrichtung nach einem der vorhergehenden Ansprüche, **dadurch gekennzeichnet, dass** der erste Verarbeitungszweig (120) eine Ultraschalleinheit (121c) umfasst, die dazu angepasst ist:
die erste Fraktion (f_{C}) aufzunehmen, und
die erste Fraktion (f_{C}) einer Ultraschallenergie auszusetzen, um die Permeabilität der Zellmembranen der Blutzellen darin mit Bezug auf mindestens ein Toxin zu erhöhen.

5. Vorrichtung nach einem der vorhergehenden Ansprüche, **dadurch gekennzeichnet, dass** der erste Verarbeitungszweig (120) eine Elektroporationseinheit (121d) umfasst, die dazu angepasst ist:
die erste Fraktion (f_{C}) aufzunehmen, und
die erste Fraktion (f_{C}) einem elektrischen Feld auszusetzen, um die Permeabilität der Zellmembranen der Blutzellen in der ersten Fraktion (f_{C}) mit Bezug auf mindestens ein Toxin zu erhöhen.

6. Vorrichtung nach einem der vorhergehenden Ansprüche, **dadurch gekennzeichnet, dass** der erste Verarbeitungszweig (120) eine Chemotherapieeinheit (121e) umfasst, die dazu angepasst ist:
die erste Fraktion (f_{C}) aufzunehmen, und
die erste Fraktion (f_{C}) einem Krebsbehandlungsmedikament auszusetzen, um eine Anzahl an krebszerstörten Zellen unter den Blutzellen in der ersten Fraktion (f_{C}) zu reduzieren.

7. Vorrichtung nach einem der vorhergehenden Ansprüche, **dadurch gekennzeichnet, dass** der erste Verarbeitungszweig (120) eine Gaszufuhreinheit (121f) umfasst, die dazu angepasst ist:
die erste Fraktion (f_{C}) aufzunehmen, und
der ersten Fraktion (f_{C}) Kohlendioxid zuzuführen, um einen intrazellulären pH-Wert der Blutzellen in der ersten Fraktion (f_{C}) zu verändern, um eine effiziente Entfernung von urämischen Toxinen zu ermöglichen.

8. Vorrichtung nach einem der vorhergehenden Ansprüche, **dadurch gekennzeichnet, dass** der zweite Verarbeitungszweig (130) eine Dialyseeinheit (132a) umfasst, die dazu angepasst ist, die zweite gereinigte Fraktion (f_{Pc}) basierend auf mindestens einem von einem Hämodialyseverfahren, einem Hämofiltrationsverfahren und einem Hämodifiltrationsverfahren zu produzieren.

9. Vorrichtung nach einem der vorhergehenden Ansprüche, **dadurch gekennzeichnet, dass** der zweite Verfahrenszweig (130) eine Säurespiegeleinstelleinheit (131a) umfasst, die dazu angepasst ist, den pH-Wert der zweiten Fraktion (f_{P}) wesentlich zu senken.

10. Vorrichtung nach Anspruch 9, **dadurch gekennzeichnet, dass** die Säurespiegeleinstelleinheit (131a) dazu angepasst ist, den pH-Wert der zweiten Fraktion (f_{P}) auf einen Wert zu senken, der in ein Intervall von ungefähr 3 bis ungefähr 5 fällt.

11. Vorrichtung nach einem der vorhergehenden Ansprüche, **dadurch gekennzeichnet, dass** der zweite Verfahrenszweig (130) eine Säurespiegeleinstelleinheit (131a) umfasst, die dazu angepasst ist, den pH-Wert der zweiten Fraktion (f_{P}) auf einen Wert zu erhöhen, der in ein Intervall von ungefähr 8 bis ungefähr 12 fällt.

12. Vorrichtung nach einem der vorhergehenden Ansprüche, **dadurch gekennzeichnet, dass** der zweite Verarbeitungszweig (130) eine zweite Medikamenteneinführeinheit (131b) umfasst, die dazu angepasst ist, der zweiten Fraktion (f_{P}) mindestens eine Eingabesubstanz hinzuzufügen, um eine resultierende vorverarbeitete zweite Fraktion (f_{Pp}) zu produzieren.

13. Vorrichtung nach einem der vorhergehenden Ansprüche, **dadurch gekennzeichnet, dass** der zweite Verarbeitungszweig (130) eine Lichtverarbeitungseinheit (131c) umfasst, die dazu angepasst ist:
die zweite Fraktion (f_{P}) aufzunehmen, und
die zweite Fraktion (f_{P}) zu beleuchten, um eine resultierende vorverarbeitete zweite Fraktion (f_{Pp}) zu erzeugen, die eine Konzentration von mindestens einem lichtempfindlichen Toxin aufweist, die geringer ist als eine Konzentration des mindestens einen lichtempfindlichen Toxins in der zweiten Fraktion (f_{P}).

14. Vorrichtung nach einem der vorhergehenden Ansprüche, **dadurch gekennzeichnet, dass** der zweite Verarbeitungszweig (130) eine Adsorptionseinheit (132b) umfasst, die dazu angepasst ist:
die zweite Fraktion (f_{P}) oder eine vorverarbeitete Fraktion davon (f_{Pp}) aufzunehmen, und
mindestens ein Toxin in dem Plasma zu adsorbieren, um die zweite gereinigte Fraktion (f_{Pc}) zu produzieren.

15. Vorrichtung nach Anspruch 14, **dadurch gekennzeichnet, dass** die Adsorptionseinheit (132b) mindestens eine positiv geladene Adsorptionszone umfasst, die dazu angepasst ist, negativ geladene Toxine in dem Plasma der zweiten Fraktion (f_{P}) zu fangen.

16. Vorrichtung nach einem der Ansprüche 14 oder 15, **dadurch gekennzeichnet, dass** die Adsorptionseinheit (132b) mindestens eine Adsorptionssäule umfasst, die dazu angepasst ist, die Konzentration des mindestens einen Toxins in dem Plasma der zweiten Fraktion (f_{P}) zu reduzieren.

17. Vorrichtung nach einem der vorhergehenden Ansprüche, **dadurch gekennzeichnet, dass** der zweite Verarbeitungszweig (130) eine Elektrodialyseeinheit (132c) umfasst, die eine Dialysemembran umfasst, wobei die Elektrodialyseeinheit (132c) dazu angepasst ist:
die zweite Fraktion (f_{P}) oder eine vorverarbeitete Fraktion davon (f_{Pp}) aufzunehmen, und
die Dialysemembran bei einer elektrischen Spannung zu halten, um geladene Substanzen aus dem Plasma zu entfernen und die zweite gereinigte Fraktion (f_{Pc}) zu produzieren.

18. Vorrichtung nach einem der vorhergehenden Ansprüche, **dadurch gekennzeichnet, dass** der zweite Verarbeitungszweig (130) eine Heizeinheit (131d) umfasst, die dazu angepasst ist:
die zweite Fraktion (f_{P}) aufzunehmen, und
die Temperatur des Plasmas zu erhöhen, um eine Anzahl an Bakterien darin zu reduzieren und die zweite gereinigte Fraktion (f_{Pc}) zu produzieren.

19. Vorrichtung nach einem der vorhergehenden Ansprüche, **dadurch gekennzeichnet, dass** der zweite Verarbeitungszweig (130) eine Antioxidanszufuhreinheit (131e) umfasst, die dazu angepasst ist:
die zweite Fraktion (f_{P}) aufzunehmen, und
dem Plasma mindestens eine Antioxidanssubstanz zuzuführen, um eine Anzahl an freien Radikalen darin zu reduzieren und die zweite gereinigte Fraktion (f_{Pc}) zu produzieren.

## Revendications

1. Appareil d'hémodialyse extracorporelle comprenant :
une unité de séparation (110) conçue pour recevoir du sang entrant (B_{A}) et à partir de ce sang diviser une première fraction (f_{C}) contenant principalement des cellules sanguines, et diviser une seconde fraction (f_{P}) contenant principalement du plasma sanguin□;
une première branche de traitement (120) conçue pour recevoir la première fraction (f_{C}), traiter la première fraction (f_{C}) en fonction d'un premier processus de dialyse et sortir une première fraction dialysée (f_{Cc}) ;
une seconde branche de traitement (130) conçue pour recevoir la seconde fraction (f_{P}), traiter la seconde fraction (f_{P}) et sortir une seconde fraction dialysée (f_{Pc})□; et
une unité de mélange (140) conçue pour recevoir les première et seconde fractions dialysées (f_{Cc}, f_{Pc}), combiner ces fractions (f_{Cc}, f_{Pc}) et sortir le sang total dialysé (B_{V}), dans laquelle la seconde branche de traitement (130) comprend au moins une seconde unité de traitement (131, 132) conçue pour éliminer des toxines liées sur les protéines dans le plasma et/ou des toxines dissoutes dans le plasma en fonction d'un second processus de dialyse différent du premier processus de dialyse,
**caractérisé en ce que**
la première branche de traitement (120) comprend au moins une première unité de traitement (121, 122) conçue pour éliminer des substances liées aux cellules présentes dans les cellules sanguines, et pour produire des cellules sanguines dialysées (f_{Cc}) ainsi obtenues.

2. Appareil selon la revendication 1, **caractérisé en ce que** la première branche de traitement (120) comprend une unité de convection (121 a) conçue pour éliminer les substances liées aux cellules de l'intérieur des cellules sanguines de la première fraction (f_{C}) au moyen d'un processus de dialyse basé sur la concentration fonctionnant sur les membranes cellulaires.

3. Appareil selon l'une quelconque des revendications 1 ou 2, **caractérisé en ce que** la première branche de traitement (120) comprend une première unité (121b) d'introduction de médicament conçue pour ajouter au moins une substance d'entrée à la première fraction (f_{C}) de façon à produire une première fraction prétraitée (f_{Cp}) ainsi obtenue.

4. Appareil selon l'une quelconque des revendications précédentes, **caractérisé en ce que** la première branche de traitement (120) comprend une unité ultrasonore (121c) conçue pour :
recevoir la première fraction (f_{C}), et
exposer la première fraction (f_{C}) à une énergie ultrasonore de façon à augmenter la perméabilité des membranes cellulaires des cellules sanguines en son sein par rapport à au moins une toxine.

5. Appareil selon l'une quelconque des revendications précédentes, **caractérisé en ce que** la première branche de traitement (120) comprend une unité d'électroporation (121d) conçue pour :
recevoir la première fraction (f_{C}), et
exposer la première fraction (f_{C}) à un champ électrique de façon à augmenter la perméabilité des membranes cellulaires des cellules sanguines dans la première fraction (f_{C}) par rapport à au moins une toxine.

6. Appareil selon l'une quelconque des revendications précédentes, **caractérisé en ce que** la première branche de traitement (120) comprend une unité de chimiothérapie (121e) conçue pour :
recevoir la première fraction (f_{C}), et
exposer la première fraction (f_{C}) à un médicament anticancéreux de façon à réduire une quantité de cellules endommagées par un cancer parmi les cellules sanguines dans la première fraction (f_{C}).

7. Appareil selon l'une quelconque des revendications précédentes, **caractérisé en ce que** la première branche de traitement (120) comprend une unité d'induction de gaz (121f) conçue pour :
recevoir la première fraction (f_{C}), et
induire du dioxyde de carbone dans la première fraction (f_{C}) de façon à modifier un niveau intracellulaire de pH des cellules sanguines dans la première fraction (f_{C}) afin de permettre une élimination efficace des toxines urémiques.

8. Appareil selon l'une quelconque des revendications précédentes, **caractérisé en ce que** la seconde branche de traitement (130) comprend une unité de dialyse (132a) conçue pour produire la seconde fraction dialysée (f_{Pc}) sur la base d'au moins un parmi un processus d'hémodialyse, un processus d'hémofiltration et un processus d'hémodiafiltration.

9. Appareil selon l'une quelconque des revendications précédentes, **caractérisé en ce que** la seconde branche de traitement (130) comprend une unité d'ajustement de niveau acide (131a) conçue pour abaisser le niveau de pH de la seconde fraction (f_{P}) de manière significative.

10. Appareil selon la revendication 9, **caractérisé en ce que** l'unité d'ajustement de niveau acide (131a) est conçue pour abaisser le niveau de pH de la seconde fraction (f_{P}) à un niveau inscrit dans un intervalle allant d'environ 3 à environ 5.

11. Appareil selon l'une quelconque des revendications précédentes, **caractérisé en ce que** la seconde branche de traitement (130) comprend une unité d'ajustement de niveau acide (131a) conçue pour augmenter le niveau de pH de la seconde fraction (f_{P}) à un niveau inscrit dans un intervalle allant d'environ 8 à environ 12.

12. Appareil selon l'une quelconque des revendications précédentes, **caractérisé en ce que** la seconde branche de traitement (130) comprend une seconde unité (131b) d'introduction de médicament conçue pour ajouter au moins une substance d'entrée à la seconde fraction (f_{P}) de façon à produire une seconde fraction prétraitée (f_{Pp}) ainsi obtenue.

13. Appareil selon l'une quelconque des revendications précédentes, **caractérisé en ce que** la seconde branche de traitement (130) comprend une unité de traitement à la lumière (131c) conçue pour :
recevoir la seconde fraction (f_{P}), et
éclairer la seconde fraction (f_{P}) afin de générer une seconde fraction prétraitée (f_{Pp}) ainsi obtenue ayant une concentration d'au moins une toxine photosensible qui est inférieure à une concentration de l'au moins une toxine photosensible dans la seconde fraction (f_{P}).

14. Appareil selon l'une quelconque des revendications précédentes, **caractérisé en ce que** la seconde branche de traitement (130) comprend une unité d'adsorption (132b) conçue pour :
recevoir la seconde fraction (f_{P}) ou une fraction prétraitée (f_{Pp}) de celle-ci, et
adsorber au moins une toxine dans le plasma de façon à produire la seconde fraction dialysée (f_{Pc}).

15. Appareil selon la revendication 14, **caractérisé en ce que** l'unité d'adsorption (132b) comprend au moins une zone d'adsorption chargée positivement conçue pour capturer des toxines chargées négativement dans le plasma de la seconde fraction (f_{P}).

16. Appareil selon l'une quelconque des revendications 14 ou 15, **caractérisé en ce que** l'unité d'adsorption (132b) comprend au moins une colonne d'adsorption conçue pour réduire la concentration d'au moins une toxine dans le plasma de la seconde fraction (f_{P}).

17. Appareil selon l'une quelconque des revendications précédentes, **caractérisé en ce que** la seconde branche de traitement (130) comprend une unité d'électrodialyse (132c) comprenant une membrane de dialyse, l'unité d'électrodialyse (132c) étant conçue pour :
recevoir la seconde fraction (f_{P}) ou une fraction prétraitée (f_{Pp}) de celle-ci, et
maintenir la membrane de dialyse à une tension électrique de façon à éliminer les substances chargées présentes dans le plasma, et produire la seconde fraction dialysée (f_{Pc}).

18. Appareil selon l'une quelconque des revendications précédentes, **caractérisé en ce que** la seconde branche de traitement (130) comprend une unité chauffante (131d) conçue pour :
recevoir la seconde fraction (f_{P}), et
élever la température du plasma de façon à réduire une quantité de bactéries en son sein, et produire la seconde fraction dialysée (f_{Pc}).

19. Appareil selon l'une quelconque des revendications précédentes, **caractérisé en ce que** la seconde branche de traitement (130) comprend une unité induisant un antioxydant (131e) conçue pour :
recevoir la seconde fraction (f_{P}), et
induire au moins une substance antioxydante dans le plasma de façon à réduire une quantité de radicaux libres en son sein, et produire la seconde fraction dialysée (f_{Pc}).
